# EUROPEAN PATENT SPECIFICATION

(11) **EP 2 717 957 B1**
(45) Date of publication and mention of the grant of the patent: **31.01.2018**
(21) Application number: 12732558.7
(22) Date of filing: 06.06.2012
(51) Int. Cl.: A61B 17/34, A61B 17/00

(54) **ARTERIAL CANNULA FOR CARDIAC SURGERY**
ARTERIELLE KANÜLE FÜR HERZCHIRURGIE
CANULE ARTÉRIELLE POUR CHIRURGIE CARDIAQUE

(30) Priority: 07.06.2011 IT FI20110116
(43) Date of publication of application: 16.04.2014
(73) Proprietor: Glauber, Mattia, 54038 Montignoso (IT)
(72) Inventor: Glauber, Mattia, 54038 Montignoso (IT)
(74) Representative: De Bonis, Paolo
(86) International application number: PCT/EP2012/060715
(87) International publication number: WO 2012/168306

(56) References cited:
- EP-A1- 1 704 889
- WO-A1-2008/014792
- WO-A1-2008/065646
- WO-A2-2004/037315
- WO-A2-2007/052278
- US-A1- 2002 002 376

## Description

### FIELD OF THE INVENTION

The present invention refers to the field of surgery devices and in particular to an arterial cannula for cardiac surgery, particularly suitable for mini-invasive cardiac surgery.

### STATE OF THE ART

Cardiac surgery includes the need to provide some sort of extracorporeal circulation where the blood is oxygenated as the cardiac surgeon makes surgical corrections to the patient's still heart. The state of the art has evolved to a more "minimally invasive" approach which is based on trying to reduce the size of the surgical incision made in the patient's chest from a fully opened chest at the sternum (full sternotomy) to an approach that results in a smaller incision either via a partial cut of the sternum (mini-sternotomy) or an approach between the ribs (mini thoracotomy) depending on the pathology. There are a number of documented benefits to the patient, however the resulting field of visualization is always much smaller as compared to that of a full sternotomy. The ability to see into the operating field is often quite limited, meaning that the instruments for this type of procedure are specialized. The cannula used in these types of procedures are also specialized, with cannula being used as a physical conduit bringing blood to and from the patient as it goes through the extracorporeal circuit. This extracorporeal circuit is used to pump, oxygenate, and temperature regulate the patient's blood during the operative course of the procedure. The point at which blood comes back into the patient is referred to as an arterial cannula, meaning the arterial blood needed by the patient's body is pumped through the arterial cannula and into the actual patient's arterial vasculature. The arterial cannulation site can be in a number of different locations including for example the ascending aorta, the aortic arch, the femoral aortic, or any other principal arteries.

Currently cannulae are not sufficiently automated for these more minimally invasive surgical procedures, because the cardiac surgeon must hold and manipulate the cannula with one hand during aortic cannulation, the other hand is often used to open the incision area, hold the tourniquet and purse strings while the cannula is being inserted into a position somewhat blind and often too far to the surgeon. For this reason, a single position retractable blade on the introducer as described in US patent 6,488,693 allows the cut and insertion of the cannula as an automated self retracting blade system; the major shortfall with this approach is that one does not know for sure if the blade has been extended or not, which poses a substantial risk in piercing the opposite wall of the vessel being cannulated. First hand experience with the commercially available device described in this patent also indicates that it is not so uneasy to push the blade too far into the cannula, dissecting the wall opposite of the cannulation site, which poses a significant risk to the patient's safety. This second cut or hole must be repaired prior to moving on with the surgical procedure, and getting to the distal cut is not particularly an easy task in a mini thoracotomy approach; this can also lead to conversion to a full sternotomy, which is what is trying to be avoided in the first place. Thus there is a need to realize a system by which the cannula/blade assembly is prevented from cutting anything but the desired location on the vessel to be cannulated.

There is also a need to help the guide of the cannula into place since visualization of the cannulation site is problematic in these minimally invasive procedures. Objective of the present invention is to provide an arterial cannula for cardiac surgery which could allow safe single wall arteria incision with minimal blood loss during insertion and removal and said cannula combined with a system that could help the guiding of the cannula and meanwhile facilitate the fixation and stabilization at the site of cannulation .

### SUMMARY OF THE INVENTION

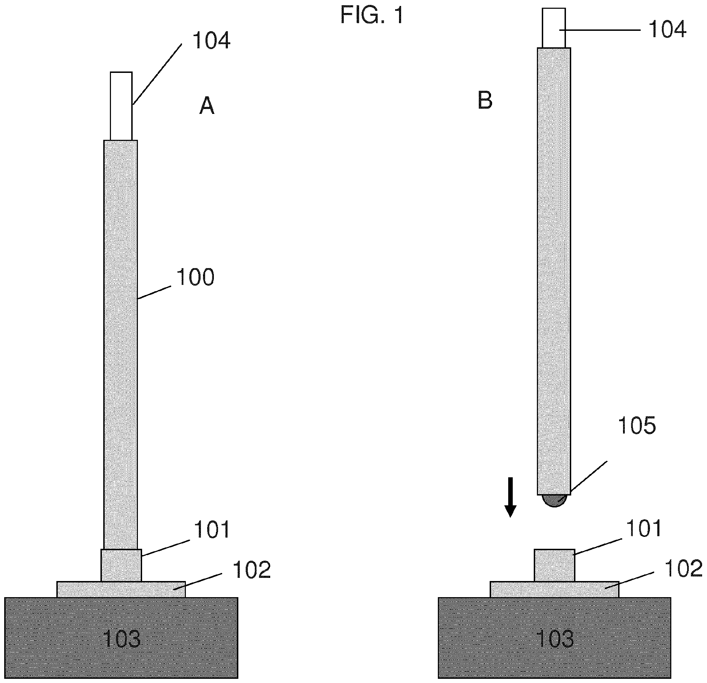

Subject matter of the present invention is, with reference to the figures 1-3, an arterial cannula (100) for cardiac surgery comprising:
(a). a pledget/stopper (102)/(101) assembly mechanically mating with the external surface of the cannula in proximity to its tip;
(b). an introducer (104), which has suitable dimension for being placed coaxially inside the cannula, said introducer having at its end a blade or a sharp edge that protrudes the cannula tip no more than 5 mm.

The cannula disclosed above is intended to facilitate the cannulation and improve the safety of aortic cannulation as compared to conventional techniques for aortic cannulation.

The use of a pledget in combination with a mechanical stopper to prevent the cannula from cutting more than the required cannulation site and well as using the pledget as a means to guide and fix the cannula into place are new and novel, and represent a significant advancement over the current state of the art, improving ease of use for the surgeon and improving the safety for the patient.

It is important to note that this approach is particularly well suited for a minimally invasive approach but it can be used also as standard cannula in central, full sternotomy conventional access. The principal difference will possibly be in the length, as the central or mini sternotomy version(s) will be shorter in length as opposed to the mini thoracotomy version, which by nature of the incision and the distance from the incision to the aortotomy will be significantly longer.

### BRIEF DESCRIPTION OF THE DRAWINGS

FIG. 1(A) shows the cannula (100) of the invention engaged with the stopper/pledget (101/102) assembly which is fixed on the vessel (103); also the introducer (104) is shown in the figure;
FIG. 1(B) shows the cannula/introducer (100/104) assembly, according to the invention, not yet engaged with the stopper/pledget (101/102) assembly already fixed at the vessel (103) to be cannulated; the blade or sharp edge of the introducer, protruding from the tip of the cannula is visible in this figure;

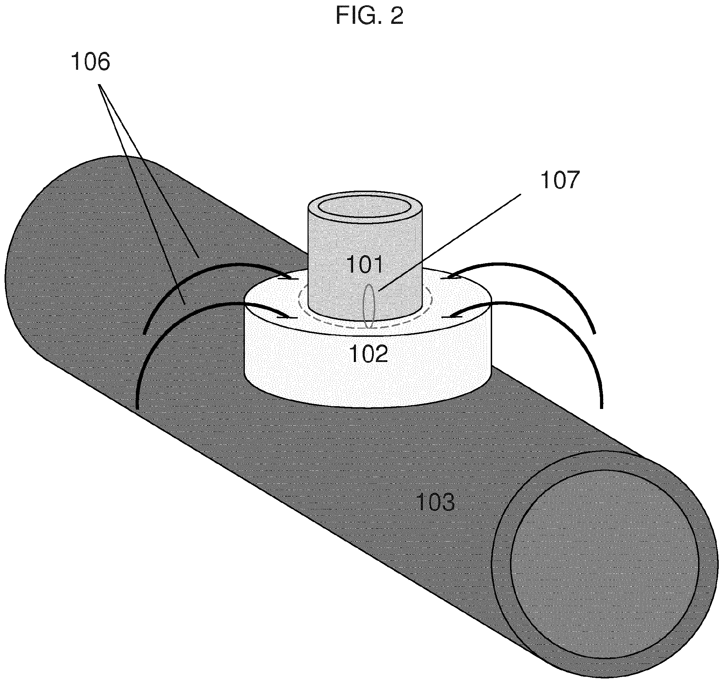

FIG.2 shows a detail of the stopper/pledget (101/102) assembly already fixed at the vessel (103) to be cannulated; in the embodiment shown in this figure the pledget is secured to the vessel by means of a minimum of two suture lines (106) and the stopper is engaged to the pledget by means of a suture with an exposed loop (107);

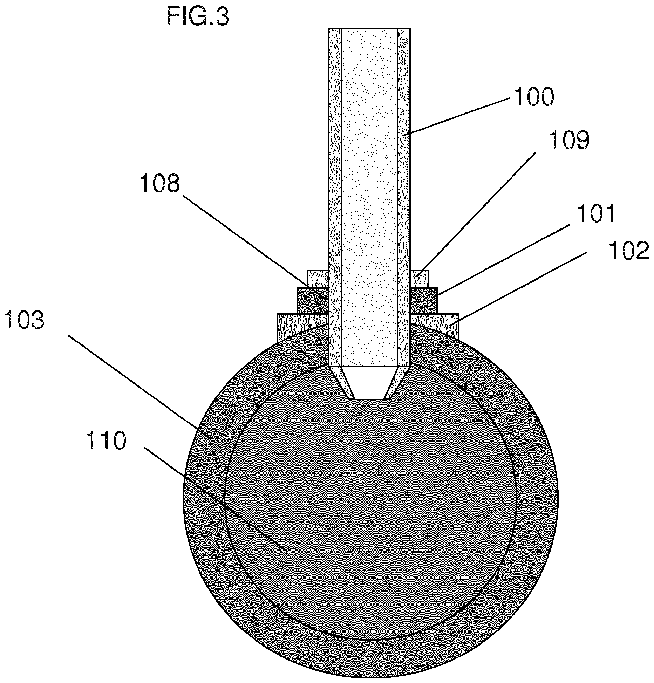

FIG. 3 shows the detail of the cannula (100), according to the invention, when it is in place into the vessel (103) and it is mechanically engaged to the pledget/stopper (101/102) assembly by means of a mechanical joint (108) and it is further secured, for not protruding to far into the vessel, by means of a molded feature (109) which, stopping against the stopper, permits the cannula to enter the aorta only to a fixed dept;

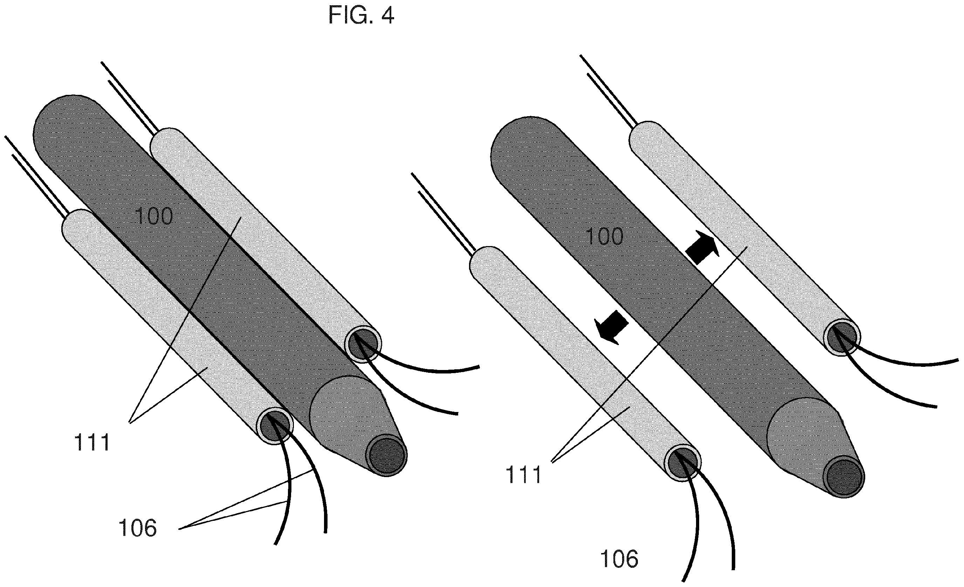

FIG. 4 shows tourniquets (111) removably attached to the external body of the cannula (100); the suture lines (106), which secures the pledget/stopper (101/102) assembly to the vessel can be introduced into said tourniquets in order to guide the cannula exactly to the cannulation site predisposed by the pledget/stopper (101/102) assembly.

### DETAILED DESCRIPTION OF THE INVENTION

The cannula according to the invention includes three separate components, the cannula body (100), the pledget and stopper assembly (101/102), and the introducer (104).

The cannula shape and its dimensions are preferably according to those already known in the art.

The introducer (104) is a semi rigid tube placed coaxially inside the cannula to facilitate cannula placement and can include a hole for wire introducer commonly used to guide the introducer and cannula assembly to the required location. The introducer can be made from a semi-rigid material such as a polyolephin or other plastic that will assist in guiding the cannula assembly to the designated cannulation site. Specific to this embodiment, the introducer includes a sharp end that protrudes past the cannula tip, either the material itself being sharp enough to piece the vessel at the cannulation site or a small blade 1 to 3 mm in depth could be included in the introducer tip to permit simple and easy vessel penetration at the cannulation site.

The tip of the cannula can be designed in any shape or manner desired to reduce the damage to the blood or improve the flow out of the cannula, in this embodiment the minimum recommended tip depth into the vessel is 10 mm. The tip of the cannula has a diameter smaller than the introducer in order to avoid that the introducer blade or sharp edge could protrude outside the cannula tip more than 1-5 mm, more preferably 1-3 mm.

One of the essential features of the present invention is that the pledget and stopper assembly (101/102) mates, snaps or otherwise interfaces with the external body of the cannula, engaged in a mechanical joint (108) based on interference or a ball-detent mechanism or male/female joint similar to that found on an ink pen cap. The stopper prevents the cannula and introducer assembly from moving too deeply into the cannulation site beyond a point established by the design of the stopper, and acts as a safety mechanism preventing accidental puncture of the opposite wall of the blood vessel being cannulated. Furthermore the external cannula body could present a molded feature (109) that permits the cannula to enter the aorta to a fixed depth, no more (the maximum recommended tip depth into the vessel is 15 mm). The molded feature (109) is a, further to the mechanical engagement (108), second secure stopper at the stopper.

The resulting cannulation is done in an inherently safer way than compared to similar embodiments as described in US patent 6,488,693 by Gannoe, et al.

The introducer and cannula assembly piece the vessel to a fixed depth established by the design of the stopper. Once the cannula is in place in the stopper, the introducer is withdrawn and thrown away. The introducer is withdrawn in a hemostatic manner, meaning that the cannula fills with blood as the introducer is withdrawn, and when the introducer is almost completely out of the cannula, the surgeon then clamps the proximal end of the cannula, preventing blood from coming out of the cannula.

The pledget-stopper assembly (101 and 102) are sutured to the vessel to be cannulated (103) and is used to guide the cannula and introducer (100 and 104), with the cannulation site being at the center of the pledget-stopper assembly . The pledget can made in such a way that once the cannula and stopper are extracted, the pledget (102) remains connected to the vessel (103).

The pledget-stopper assembly may include a minimum of two suture lines (106) that are used to fix the pledget to the vessel to be cannulated; these suture lines can be included in the assembly or may be fixed onto the pledget by the surgeon manually. The stopper can be made of any material, preferably a polycarbonate or other moldable plastic that interfaces mechanically with the tip of the cannula as described above. The pledget is made from typical pledget/bandage material, like a sturdy wool or other bandage material that is flexible enough to take the shape of the curvature of the vessel (103) to which it is attached. The sutures included on the pledget are used to sew the assembly to the vessel. A minimum of two sutures with needles included at each end of the suture line are preferable but more than two lines can easily be included as an improvement or other embodiment of the invention.

As the cannula and connected stopper are removed from the cannulation site, the pledget is then used to close the hole left behind in the vessel (i.e. cannulation site) preventing blood loss. This is a very advantageous additional feature of the invention because the surgeon often does not have clear visible access to the aorta and cannulation site in a MICS mini thoracotomy or mini sternotomy approach. Being able to leave a pledget on the vessel and using the suture lines (106) to close the vessel, as described below, is therefore highly advantageous due to this lack of visualization.

Therefore the pledget and stopper are preferably each other joined mechanically in a manner that will permit easy detachment of the stopper once the procedure is complete and the cannula needs to be removed. One embodiment could be using a suture with an exposed loop (107) joining the stopper to the pledget; the exposed loop could be cut, breaking the suture which joins the stopper to the pledget, so that the suture line could be extracted, freeing the stopper from the pledget. The stopper remains attached to the cannula and is extracted along with the cannula when the assembly is removed, while the pledget remains attached to the vessel. By crossing the sutures (106) the vessel can be easily closed when the sutures are crossed and tied off manually with a knot or using a more sophisticated tie off technique, permitting simple closure of the cannulation site.

Again, at least two suture lines would be required, however any number more than two could be realized in this design.

In many cases the cannulation site is not entirely visible, so a method for aligning the central axis of the stopper with the tip of the cannula is highly advantageous.

Such a technique can permit the proper landing of the cannula tip to the seat of the pledget stopper assembly, and can be provided quite easily by the use of the suture strings (106) and a mechanical guide on the body of the cannula which permits the use of the suture strings to guide the tip of the cannula directly into the stopper. These same suture strings (106) can then be placed into a tube (typically called a tourniquet (111) and mechanically attached diametrically opposed to the external surface of the cannula. This allows the tourniquet assembly with the suture lines inside to be safely secured out of the field of view, which is important particularly if the surgical incision and consequent surgical field is quite small.

## Claims

1. An arterial cannula (100) for cardiac surgery comprising
an introducer (104), which has suitable dimension for being placed coaxially inside the cannula, said introducer having at its end a blade or a sharp edge that protrudes the cannula tip no more than 5 mm, the arterial cannula (100) being **characterized in that**
the cannula (100) further comprises a pledget and stopper (101/102) assembly mechanically mating with the external surface of the cannula in proximity to its tip,
whereby the pledget and stopper assembly (101/102) mates with the external body of the cannula, engaged in a mechanical joint (108) that is based on interference or a ball-detent mechanism or male/female joint.

2. Arterial cannula according to claim 1 comprising at its external surface a molded feature (109) that is, further to the mechanical engagement (108), a second secure stopper at the stopper (102).

3. Arterial cannula according to any of claims 1-2 wherein the pledget-stopper assembly include a minimum of two suture lines (106) that are used to fix the pledget to the vessel to be cannulated.

4. Arterial cannula according to any of claims 1-3 wherein the pledget and stopper are each other joined mechanically in a manner that will permit easy detachment of the stopper once the procedure is complete and the cannula needs to be removed.

5. Arterial cannula according to any of claims 1-4 comprising at least two tourniquets (111) attached diametrically opposed to the external surface of the cannula.

## Patentansprüche

1. Eine arterielle Kanüle (100) für Herzchirurgie umfassend
einen Inserter (104), der geeignete Abmessungen aufweist, um koaxial innerhalb der Kanüle angeordnet zu werden, wobei der Inserter an seinem Ende eine Klinge oder eine scharfe Kante besitzt, die nicht mehr als 5 mm über die Spitze der Kanüle vorragt, wobei die arterielle Kanüle (100) **dadurch gekennzeichnet ist,**
**dass** die Kanüle des Weiteren eine mit der äußeren Oberfläche der Kanüle in der Nähe ihrer Spitze mechanisch verbundene Pledget- und Anschlagbaugruppe (101/102) umfasst,
wobei die Pledget- und Anschlagbaugruppe (101/102) mit dem Außenkörper der Kanüle über eine mechanische Verbindung (108) in Eingriff steht, welche auf einem Übermaß oder einem Kugelrast-Mechanismus oder einer Stecker/Buchsen-Verbindung basiert.

2. Arterielle Kanüle nach Anspruch 1, die an ihrer Außenfläche eine Anformung (109) aufweist, welche zusätzlich zu der mechanischen Verbindung (108) einen zweiten Sicherheitsanschlag an dem Anschlagelement (102) bildet.

3. Arterielle Kanüle nach einem der Ansprüche 1 bis 2, **dadurch gekennzeichnet, dass** die Pledget- und Anschlagbaugruppe mindestens zwei Nahtlinien (106) beinhaltet, die verwendet werden, um das Pledget an dem zu kanulierenden Gefäß zu befestigen.

4. Arterielle Kanüle nach einem der Ansprüche 1 bis 3, **dadurch gekennzeichnet, dass** das Pledget und das Anschlagelement miteinander mechanisch verbunden sind in einer Art, die ein einfaches Lösen des Anschlagelements erlaubt, wenn das Verfahren abgeschlossen ist und die Kanüle entfernt werden soll.

5. Arterielle Kanüle nach einem der Ansprüche 1 bis 4, umfassend mindestens zwei Tourniquets (111), die diametral entgegengesetzt an der Außenfläche der Kanüle angebracht sind.

## Revendications

1. Canule artérielle (100) pour chirurgie cardiaque comprenant
un dispositif d'introduction (104), qui a une dimension appropriée pour être placé de manière coaxiale à l'intérieur de la canule, ledit dispositif d'introduction ayant au niveau de son extrémité une lame ou un bord tranchant qui dépasse de la pointe de canule d'au plus 5 mm, la canule artérielle (100) étant **caractérisée en ce que**
la canule (100) comprend en outre un ensemble compresse et bouchon (101/102) s'accouplant mécaniquement avec la surface externe de la canule à proximité de sa pointe,
moyennant quoi l'ensemble compresse et bouchon (101/102) s'accouple avec le corps externe de la canule, engagée dans un joint mécanique (108) qui est basé sur une interférence ou un mécanisme de détente à bille ou un joint mâle/femelle.

2. Canule artérielle selon la revendication 1, comprenant au niveau de sa surface externe un élément moulé (109) qui est, outre l'engagement mécanique (108), un deuxième bouchon de sécurité au niveau du bouchon (102).

3. Canule artérielle selon l'une des revendications 1 et 2, dans laquelle l'ensemble compresse-bouchon comporte un minimum de deux lignes de suture (106) qui sont utilisées pour fixer la compresse au vaisseau devant subir une canulation.

4. Canule artérielle selon l'une des revendications 1 à 3, dans laquelle la compresse et le bouchon sont mécaniquement reliés entre eux d'une manière qui permettra un détachement facile du bouchon une fois que la procédure est achevée et qu'il est nécessaire de retirer la canule.

5. Canule artérielle selon l'une des revendications 1 à 4 comprenant au moins deux garrots (111) attachés en étant diamétralement opposés à la surface externe de la canule.
